Europäisches Patentamt

European Patent Office

Office européen des brevets

(11) Publication number: **0 293 524**
**A1**

## (12) EUROPEAN PATENT APPLICATION

(21) Application number: 87304848.2

(22) Date of filing: 02.06.87

(51) Int. Cl.⁴ **C07K 15/00 , C12P 21/00 ,**
**A61K 39/00 , G01N 33/564 ,**
**G01N 33/68**

(43) Date of publication of application:
**07.12.88 Bulletin 88/49**

(84) Designated Contracting States:
**AT BE CH DE ES FR GB GR IT LI LU NL SE**

(71) Applicant: **VASOCOR**
**2438 Wyandotte Street**
**Mountain View California 94013(US)**

(72) Inventor: **Calenoff, Emanuel**
**1824 Oak Creek Drive, No.319**
**Palo Alto California 94304(US)**

(74) Representative: **Harrison, David Christopher**
**et al**
**MEWBURN ELLIS & CO 2/3 Cursitor Street**
**London EC4A 1BQ(GB)**

(54) Atherosclerotic plaque immunoassay.

(57) Novel reagents of this invention useful in immunoassays of patient serum to detecting evidence of the presence of atherosclerotic plaque include atherosclerotic plaque antigen, atherosclerotic plaque antigen conjugated with a distinctive label, antibody which binds preferentially to atherosclerotic plaque antigen, and antibody conjugated with a distinctive label which binds preferentially to atherosclerotic plaque antigen. These reagents can be used in a variety of competition and sandwich immunoassays.

A preferred immunoassay method for determining the presence of anti-(atherosclerotic plaque antigen) antibody in a serum sample comprises contacting the sample with an insoluble support to which atherosclerotic plaque antigen is adhered for a time sufficient to permit plaque antigen conjugation with anti-(atherosclerotic plaque antigen) antibody to occur. The insoluble support is then contacted with primary antibody which will bind with anti-(atherosclerotic plaque antigen) antibody for a sufficient time to permit conjugation to occur therebetween. The presence and amount of the primary antibody conjugated to the insoluble support is then determined.

EP 0 293 524 A1

## ATHEROSCLEROTIC PLAQUE IMMUNOASSAY

This invention relates to an immunoassay method and reagents for determining the presence of atherosclerotic plaque. In particular, the method of this invention is a serum immunoassay for determining the presence of antibodies in patient serum which bind preferentially with atherosclerotic plaque antigens.

The endothelium located between the blood and arterial tissue serves as a barrier against the accumulation of blood components in the vascular wall. Formation of arteriosclerotic lesions in the endothelium is associated with major coronary disease and stroke, and the causes and detection of such lesions has been intensely investigated.

Endothelial injury, believed to be an initial step in the formation of the atherosclerotic lesions, can be potentially caused by hemodynamic strain, hypercholesterolemia, hypertension and immune complex disease. Endothelial injury leads to intimal thickening, cell proliferation, cholesterol accumulation and formation of connective tissue fibers. IgG and complement factor C3 accumulation in injured endothelial cells and nonendothehelialized intima has been indicated. Mononuclear phagocytes derived from blood have also been found to constitute a part of the cell population in atherosclerotic lesions. The mechanisms leading to the ultimate plaque composition are as yet unknown.

A side variety of soluble proteins have been extracted from human atherosclerotic plaque, including IgA, IgG, IgM, B1C C3), alphal-antitrypsin, alpha2-macroglobulin, firbrinogen, albumin, LDL, HDL, alphal-acid glycoprotein, beta2-glycoprotein, transferrin and ceruloplasmin. The diseased intima was also found to contain a small amount of tissue-bound IgG, IgA and B1C. Hollander, W. et al. Atherosclerosis. 34:391-405 (1979). Other scientists have reported IgG in the lesions and adjacent endothelial tissue. Parums, D. et al Athrosclerosis. 38:211-216 (1981), Hansson, G. et al Experimental and Molecular Pathology. 34:264-280 (1981), Hansson, G. et al Acta Path. Microbiol. Immunol. Scand. Sect.A. 92:429-435 (1984). However, the origin, function and binding properties of the immunoglobulins in the atherosclerotic and associated tissue is still a mystery. Anti-low density lipoprotein (LDL) autoantibodies has been reported to be higher in patients of vascular disease, suggesting that they are associated in some way with arteriosclerotic manifestations. However, no causal relationship between these autoantibodies and arteriosclerotic plaque has not been established. Szondy, E. et al Mechanisms of Aging and Development. 29:117-123 (1985).

A wide variety of immunoassays have been developed for determining the presence and amount of a wide variety of antigenic and non-antigenic materials in diverse body fluids and tissues. Total immunoglobulin and IgE immunoassays are described in U.S. patents 3,720,760 and 4,444,879. IgG allotype immunoassays are described in Russian Patent 649,433. ELISA immunoassays are described by Maggio, et al. ENZYME-IMMUNOASSAY. Boca Raton: CRC Press pp 172-176 (1980). However, prior to this invention, no immunoassay suitable for determining the presence of atherosclerotic plaque has been known.

The method of this invention for determining the presence of anti-plaque antibodies in serum comprises contacting the serum with plaque antigen for a time sufficient to permit binding of the antigen with anti-plaque antibody and determining antigen binding, if any, with the anti-plaque antibody. The plaque antigen can be bound to an insoluble support, contacted with serum to permit anti-plaque antibody conjugation with the plaque antigen, and the amount of resultant antibody bound to the insoluble support can be determined. Alternatively, anti-plaque reagent antibody can be bound to an insoluble support, and a mixture of serum and labeled plaque antigen can be contacted therewith. The labeled plaque antigen bound to the insoluble support or remaining in the mixture can then be determined. In a further embodiment, the antigen can be bound to the insoluble support, and a mixture of serum and labeled anti-plaque reagent antibody can be contacted therewith. The labeled anti-plaque reagent antibody bound to the insoluble support or remaining in the mixture can then be determined.

The compositions of this invention include purified plaque antigen, labeled plaque antigen, anti-plaque antibody reagent, plaque antigen bound to an insoluble support and anti-plaque reagent antibody bound to an insoluble support.

The method of this invention for determining the presence of anti-plaque antibodies in serum comprises contacting the serum with plaque antigen, either alone or together with anti-plaque reagent antibody.

Plaque antigen is obtained by processing human arteriosclerotic plaque to isolate and purify antigen specific thereto. The plaque can be obtained by processing human aortas from patients who have died from myocardial infraction, cerebral vascular accident or can be obtained from surgical procedures. The aortae are dissected free of surrounding tissue and fat and rinsed in saline to remove contaminating blood. Segments of plaques are separated and processed.

The plaque areas are removed, cut into small pieces, washed briefly, and homogenized in isotonic solution such as phosphate-buffered saline (PBS). The homogenate repeatedly centrifuged and resuspended in PBS, the eluates being collected. The sedimented debris can also be extracted with citrate buffer, acid pH, and the neutralized eluate is neutralized and purified by dialysis. The supernatant as well as the pooled PBS eluates can be concentrated by vacuum dialysis and ultrafiltration to yield a concentrate of soluble plaque antigens.

Insoluble plaque antigens can be obtained by digesting portions with different enzymes, extracting the digest with PBS, and concentrating the eluates as described above. The enzyme compositions used can include collagenase, elastase, DNase, hyaluronidase, and neuraminidase. Each digest is centrifuged, concentrated as by ultrafiltration, for example, and dialyzed to yield a concentrated solution of insoluble plaque antigens.

Anti-plaque reagent antibody is obtained by immunizing an animal such as a rabbit, guinea pig, rat or goat with concentrated plaque antigen, removing serum from the immunized animal, and separating the immunoglobulins from the serum, for example by ammonium sulfate precipitation. The principal antibodies useful in the method of this invention are IgG and IgM antibodies, although the IgE and IgA antibodies can also be used if available in sufficient quantity.

The plaque antigen and anti-plaque reagent antibody can be bonded to an insoluble support by conventional processes. Procedures for binding of antibodies to insoluble supports are described in U.S. patents 3,551,555, 3,553,310, 4,048,298 and RE-29,474, for example. Binding of antibodies to polystyrene by adsorption has been described in U.S. patents 3,646,346 and 4,092,408, for example. Binding of protein containing antigens to a variety of insoluble supports has been described in U.S. Patent 3,720,760.

A variety of materials can be used as the insoluble support, the primary consideration being the binding of the anti-plaque antibody or the plaque antigen to the surface, the absence of interference with the anti-plaque antibody and plaque antigen conjugating reaction or with other reactions which can be employed to determine the presence and extent of the conjugating reaction. Organic and inorganic polymers, both natural and synthetic, can be used as the insoluble support. Examples of suitable polymers include polyethylene, polypropylene, polybutylene, poly(4-methylbutylene), butyl rubber, silastic polymers, polyesters, polyamides, cellulose and cellulose derivatives (such as cellulose acetate, nitrocellulose and the like), acrylates, methacrylates, vinyl polymers (such as polyvinyl acetate, polyvinyl chloride, polyvinylidene chloride, polyvinyl fluoride, and the like), polystyrene and styrene graft copolymers, rayon, nylon, polyvinylbutyrate, polyformaldehyde, etc. Other materials which can be used as the insoluble support can the latexes of the above polymers, silica gel, silicon wafers, glass, paper, insoluble protein, metals, metalloids, metal oxides, magnetic materials, semi-conductive materials, cermets and the like. In addition are included substances which form gels, such as proteins such as gelatins, lipopolysaccharides, silicates, agarose, polyacrylamides or polymers which form several aqueous phases such as dextrans, polyalkylene glycols (alkyene with 2 to 3 carbon atoms) or surfactants, e.g. amphophilic compounds such as phospholipids, long chain (12-24 carbon atoms) alkyl ammonium salts and the like.

A preferred diagnostic support of this invention comprises a polystyrene, styrene copolymers such as styrene-acrylonitrile copolymers, or polyolefins such as polyethylene or polypropylene, and acrylate and methacrylate polymers and copolymers. The anti-plaque reagent antibody or the plaque antigen can be bound to the insoluble support by adsorption, ionic bonding, van der Waals adsorption, electrostatic bonding, or other non-covalent bonding, or it can be bound to the insoluble support by covalent bonding. A particularly advantageous support for this procedure comprises a microtiter plate having a plurality of wells. The well surface or plastic cup inserts therein can constitute the antigen or antibody support. If the determination will require the use of fluorometric measurements, the microtiter plate or the well inserts are advantageously opaque to light so that excitation light applied to a well does not reach or influence contents of the surrounding wells.

Procedures for non-covalent bonding are described in U.S. Patent 4,528,267. Procedures for covalently bonding antibodies and antigens to insoluble supports are described by Ichiro Chibata in IMMOBILIZED ENZYMES. Halsted Press: New York (1978) and A. Cuatrecasas, J. Bio. Chem. 245:3059 (1970), the entire contents of which are hereby incorporated by reference. The surface can be coated with a protein and coupled with the antibody or antigen using procedures described in U.S. Patent 4,210,418 using glutaraldehyde as a coupling agent, for example. In a still further procedure, the well can be coated with a layer having free isocyanate groups such as a polyether isocyanate, and application of the antibody or antigen in aqueous solution thereto effects the requisite bonding. In a still further procedure, the antibody or antigen can be coupled to a hydroxylated material by means of cyanogen bromide as described in U.S. Patent 3,720,760.

The labeled plaque antigens and anti-plaque antibodies of this invention can be prepared by conventional procedures for attaching labels to proteins. The labels can be bonded or coupled to the protein reagents by chemical or physical bonding. Ligands and groups which can be conjugated to the antibodies

3

and plaque antigens of this invention include elements, compounds or biological materials which have physical or chemical characteristics which can be used to distinguish the reagents to which they are bonded from compounds and materials in the sample being tested. As a general principle, methods suitable for linking a label with an antibody are equally suitable for linking or bonding the label to a plaque antigen. Although the following procedures are described in terms of conjugating labels to antibodies, their use to conjugate labels to plaque antigens are also intended.

Radiolabeled plaque antigens and anti-plaque antibodies of this invention can be used for in vitro diagnostic tests. The specific activity of a tagged antibody depends upon the half-life, isotopic purity of the radioactive label and how the label is incorporated into the antigen or antibody. Table A lists several commonly used isotopes, their specific activities and half-lives. In immunoassay tests, the higher the specific activity, in general, the better the sensitivity.

## Table A

| Isotope | Specific Activity of Pure Isotope (Curies/mole) | Half-Life |
|---------|-------------------------------------------------|-----------|
| $^{14}C$ | $6.25 \times 10^1$ | 5720 years |
| $^3H$ | $2.91 \times 10^4$ | 12.5 years |
| $^{35}S$ | $1.50 \times 10^6$ | 87 days |
| $^{125}I$ | $2.18 \times 10^6$ | 60 days |
| $^{32}P$ | $3.16 \times 10^6$ | 14.3 days |
| $^{131}I$ | $1.62 \times 10^7$ | 8.1 days |

Procedures for labeling proteinaceous antigens and antibodies. with radioactive isotopes listed in Table A are generally known in the art. Tritium labeling procedures are described in U.S.Patent 4,302,438, for example. Iodinating, tritium labeling and 35S labeling procedures especially adapted for antibodies are described by Goding, J.W. MONOCLONAL ANTIBODIES: PRINCIPLES AND PRACTICE. New York: Academic Press pp 124-126 (1983) and the references cited therein. Other procedures for iodinating antibodies are described by Hunter and Greenwood, Nature. 144:945 (1962) and David et al, Biochemistry. 13:1014-1021 (1974) and in U.S.patents 3,867,517 and 4,376,110.

Radiolabeled antibodies can be also be used for imaging and potentially for radiotherapy. Radiolabeling elements which are useful in imaging include $^{123}$I, $^{131}$I, $^{111}$In, and $^{99m}$Tc, for example. Procedures for iodinating antibodies are described by Greenwood, F. et al, "The preparation of 131I-labeled human growth hormone of high specific radioactivity," Biochem.J. 89:114-123 (1963); Marchalonis, J. "An enzymatic method for the trace iodination of immunoglobulins and other proteins," Biochem.J. 113:299-305 (1969); and Morrison, M. et al. "Use of lactoperoxidase catalyzed iodination in immunochemical studies," Immunochemistry. 8:289-297 (1971). Procedures for 99mTc-labeling are described by Rhodes, B. et al. "99mTc-Labeling and acceptance testing of radiolabeled antibodies and antibody fragments," in Burchiel, S. et al (eds.), TUMOR IMAGING: THE RADIOIMMUNOCHEMICAL DETECTION OF CANCER. New York: Masson 111-123 (1982), and the references cited therein. Procedures suitable for 111In-labeling antibodies are described by Hnatowich, D.J. et al. J.Immul.Methods. 65:147-157 (1983); Hnatowich, D.J. et al. Science. 220:613-615 (1983) and Buckley, R.G. et al F.E.B.S. 166:202-204 (1984).

Antibodies labeled with enzymes are useful for in vitro diagnostic tests such as are described in United States patent application Serial No. 622,525 filed June 20, 1984. Examples of suitable systems, coupling procedures and substrate reactions therewith are disclosed in U.S. Patents Re. 31,006, B1\3,654,090, 4,214,048, 4,289,747, 4,302,438, 4,312,943, 4,376,110 and the references cited therein, for example. Examples of other suitable systems are described by Pesce et al, Clin.Chem. 20(3):353-359 (1974) and Wisdom, G., Clin.Chem. 22:1243 (1976).

A list of suitable enzyme classes and specific examples for each class follow:

## TABLE I

| Class | Enzyme Example |
|---|---|
| Hydrolases Carbohydroases | Amylases |
| Nucleases | Polynucleotidase |
| Amidases | Arginase |
| Purine deaminases | Adenase |
| Peptidases | Aminopolypeptidase |
| Proteinases | Pepsin |
| Esterases | Lipases |
| Iron Enzymes | Catalase |
| Copper Enzymes | Tyrosinases |
| Enzymes containing Coenzymes | Alcohol dehydrogenase |
| Enzymes reducing cytochrome | Succinic dehydrogenase |
| Yellow enzymes . | Diaphorase |
| Mutases | Glyoxalase |
| Demolases | Aldolase |
| Oxidases | Glucose oxidase |
| | Horse radish peroxidase |
| Other enzymes | Beta-galactosidase |
| | Phosphatases |
| | Phosphorylases |
| | Hexokinases |

A list of suitable enzymes are described in Hawk, et al. PRACTICAL PHYSIOLOGICAL CHEMISTRY, New York: McGraw-Hill pp 306-397 (1954).

Fluorogenic enzymes (enzymes in the presence of which a selected substrate will produce a fluorescent product) are useful labeling moieties. Methods for selectively conjugating enzymes to antibodies without impairing the ability of the antibody to bind with antigen are well known in the art. Suitable enzymes and procedures for coupling them to antibodies are described by Wilson, M. et al. Recent developments in the periodate method for conjugating horseradish peroxidase (HRPO) to antibodies. INTERNATIONAL CONFERENCE IN IMMUNOFLUORESCENCE AND RELATED STAINING TECHNIQUES. W. Knapp et al, editors. Amsterdam: Elsevier pp 215-244 (1978), Sullivan, M. et al. Enzyme immunoassay: a review. Annals of Clinical Biochemistry. 16:221-240 (1979) and in U.S. Patent 4,190,496, for example. The preferred fluorogenic enzymes and suitable substrates corresponding thereto include horseradish peroxidase for which a suitable substrate is homovanillic acid or 4-hydroxy-3-methoxy-phenylacetic acid, beta-galactosidase for which a suitable substrate is 4-methylumbelliferylbeta-D-galactoside, alkaline phosphatase for which a suitable substrate is 4-methylumbelliferyl phosphate and other umbelliferyl phosphates such as 4-carboxyumbelliferyl phosphate and umbelliferyl phosphate 4-carboxyalkyl-esters, etc.

Examples of suitable procedures for enzyme labeling the antibody include the use of carbodiimides, dialdehydes, and bifunctional coupling reagents. Linkage of enzymes through amide groups can be achieved by treating the proteins with thionyl chloride, N-hydroxysuccinimide or similar reagents in an anhydrous solvent such as dimethylformamide, dioxane, dimethylsulfoxide, tetrahydrofuran, or the like. Alternative coupling agents include carbodiimides such as 1-ethyl-3-(3-N,N',-dimethylaminopropyl)-

carbodiimide or 1-cyclohexyl-3-(2-morpholinoethyl)carbodiimide methyl-p-toluenesulfonate.

The carbohydrate moiety; of an enzyme can also be oxidized to an aldehyde and reacted with lysyl amino group of immunoglobulins to form a Schiffs base. Reduction with sodium borohydride effects a stable linkage of enzyme and antibody. Horseradish peroxidase with antibody can be efficiently linked to immunoglobulins by the method of Wilson, supra.

Fluorescent labeled antibodies can be prepared from standard fluorescent moieties known in the art. Since antibodies and other proteins absorb light having wavelengths up to about 310 nm, the fluorescent moieties should be selected to have substantial absorption at wavelengths above 310 nm and preferably above 400 nm. A variety of suitable fluorescers are described by Stryer, Science. 162:526 (1968) and Brand, L. et al, "Fluorescent probes for structure," Annual Review of Biochemistry. 41:843-868 (1972). The antibodies can be labeled with fluorescent groups by conventional procedures such as those disclosed in U.S. Patents 3,940,475, 4,289,747 and 4,376,110, for example.

One group of fluorescers having a number of the desirable properties described above are the xanthene dyes, which include the fluoresceins derived from 3,6-dihydroxy-9-phenylxanthhydrol and resamines and rhodamines derived from 3,6-diamino-9-phenylxanthydrol and lissanime rhodamine B. The rhodamine and fluorescein derivatives of 9-o-carboxyphenylxanthhydrol have a 9-o-carboxyphenyl group. Fluorescein compounds having reactive coupling groups such as amino and isothiocyanate groups such as fluorescein isothiocyanate and fluorescamine are readily available.

Another group of fluorescent compounds are the naphthylamines, having an amino group in the alpha or beta position. Included among the naphthylamino compounds are 1-dimethylaminonaphthyl-5-sulfonate, 1-anilino-8-naphthalene sulfonate and 2-p-toluidinyl-6-naphthalene sulfonate. Other dyes include 3-phenyl-7-isocyanatocoumarin; acridines such as 9-isothiocyanatoacridine and acridine orange;
N-[p-(2-benzoxazolyl)phenyl]maleimide; benzoxadiozoles such as 4-chloro-7-nitrobenzo-2-oxa-1,3-diazole and 7-(p-methoxybenzylamino)-4-nitrobenzo-2-oxa-1,3-diazole; stilbenes such as
4-dimethylamino-4'-isothiocyanatostilbene and 4-dimethylanimo-4'-maleimidostilbene;
N,N',-dioctadecycloxacarboxyamine-p-toluenesulfonate;
pyrenes such as 8-hydroxy-1,3,6-pyrenetrisulfonic acid, 1-pyrenebutyric acid, merocyanine 540, rose bengal, 2,4-diphenyl-3(2H)-furanone, o-phthaldehyde, as well as other readily available fluorescing molecules. These dyes either have active functionalities or such functionalities can be readily introduced.

For example, antibodies can be labeled with fluorochromes by the procedures described by Goding, J. MONOCLONAL ANTIBODIES: PRINCIPLES AND PRACTICE. New York: Academic Press, pp 208-249 (1983). The concentration of fluorochrome is selected according to the table of Goding, p 229. For example, fluorescein isocyanate (1.0 mg/ml) or rhodamine isocyanate (10.0 mg/ml) in DMSO is prepared, and the desired volume (1-10% of total protein solution volume) is added to the protein solution dropwise, with stirring. The reaction proceeds for two hours, shielded from light. The product is purified by gel filtration on SEPHADEX G-25 gel in PBS containing 0.1% NaN3 to separate the unreacted or hydrolyzed fluorochrome. The absorbance of the conjugate is measured at 280 nm and at its peak in the visible region (495 nm for fluoresceinated antibody and 550 nm for rhodaminated antibody). The fluorochrome to protein ratio is calculated according to the procedure of Goding, supra, p 224-225. Conjugates are stored at 4C protected from light until use. If the antibody solution concentration is less than 1 mg/ml, BSA is added to the solution to a final concentration of 1 mg/ml.

The antibodies of this invention can be covalently bonded to avidin or biotin in one embodiment of this invention. Suitable binding procedures involve cross-linking through a bifunctional cross-linking agent. Suitable bifunctional compounds are described by Peters, K. et al Ann.Rev.Biochim. 46:523 (1977). Alkyl imidates show a high degree of specificity among the functional groups presented to them by a protein. The reaction is specific for primary amino groups. Examples of suitable coupling reagents include amidoesters such as dimethylmalonimidate, azides such as the acyl azide of tartryl diazide which reacts readily with immuno groups to produce amide linkages. Aryl dihalides (e.g., 1,5-difluoro-2,4-dinitrobenzene, or
4,4',-difluoro-3,3',-dinitrophenyl sulfone, glutaraldehyde,
1-ethyl-3-(3-dimethylaminopropyl)carbodiimide hydrochloride, dimaleinide, mixed anhydride,
m-maleamidobenzoyl N-hydroxysucciinimide ester, and other known cross-linking agents.

The foregoing reagents provide essentially irreversible bonds. Bifunctional agents with functional groups such as disulfide or glycol may be used. These provide bonds which can be broken after the cross-linking reaction, if desired. Such reagents include dimethyl 3,3',-dithiobispropionimidate, succinimidyl propionimidate, N-(3-fluoro-4,6-dinitrophenyl)-cystamine, tartryl diazide, tartryl di(glycylazide) and tartryl di-(epsilon-amino caproylazide).

In other instances, the bonds can be formed directly between the reagents themselves. For example, antibody can be bound to biotin through functional groups on the respective materials. As a specific example, biotin can be treated with periodate and reacted with antibody to give a Schiff base formation without inhibiting the biotin to avidin binding or blocking immunological activity of the antibody.

Known techniques using bifunctional cross-linking agents include the following: (a) a one-step glutaraldehyde linkage, Avrameas, S., Immunochemistry. 6:43 (1969); (b) two-step glutaraldehyde linkage, Avrameas, S., Immunochemistry. 8:1175 (1971); and (c) dimaleimide linkage, Kato, K. et al, Euro.J.Biochem. 62:285 (1966).

Antibodies can be labeled with metallic radionuclides according the procedure of Hnatowich, D. et al. Journal of Applied Radiation. 35(6):554-557 (1984) and Buckley, R et al. Federation of European Biochemical Societies. 166(1):202-204 (Jan. 1984). In this procedure the antibodies are conjugated with a chelating agent such as diethylenetriamine pentaacetic acid which is capable of forming a chelate with the metallic radionuclide. A suspension of 0.1 mg/ml of the bicyclic anhydride of DTPA (diethylenetriamine pentaacetic acid) is prepared in a dry solvent such as chloroform, ether or dry DMSO. An aliquot is removed to a clean, dry tube sufficient to provide a DTPA to immunoglobulin molar ratio of 1:1 and evaporated under nitrogen. A 10-20 microliter portion of the antibody solution used (10-20 mg/ml) in 0.05M bicarbonate buffer in saline, pH 7.0-7.5 is added to the dry DTPA, and the contents are agitated for 0.5-1.0 min. The coupled protein preparation is diluted to 0.2 ml with the same buffer solution and purified on a 5 cm gel filtration column with SEPHADEX G-50 gel, using a saline eluant. The coupling efficiency is determined before purification by the addition of "chelation-grade" 111In in 0.5M acetate buffer solution, pH 6.0. Thin layer chromatograpy is used to separate the DTPA coupled antibody for calculation of the coupling efficiency. The DTPA-coupled antibodies can be stored at 4C until needed for binding with metallic radionuclides such as $^{111}In^{+3}$, $^{212}Bi^{+3}$ and $^{68}Ga^{+3}$, for example.

In the sandwich assay methods of this invention, an insoluble to which plaque antigen is adhered is contacted with patient serum for a sufficient time to permit conjugation of anti-plaque antibody with the plaque antigen and then removing the patient serum from the support. The incubation time should be sufficient to permit substantial conjugation to occur, the time being temperature dependent. Suitable incubation times are from 30 to 240 minutes at temperatures within the range of from 16 to 40C, the preferred contact time being at least 60 minutes at temperatures within the range of from 20 to 26C.

The residual serum is then removed form the support by use of a rinse solution. Any conventional rinse solution can be used. A preferred rinse solution is described in U.S.Patent 4,528,267. It is an aqueous phosphate buffer solution having a phosphate molarity of from 0.0001 to 0.05, a pH of from 6 to 8 and containing from 0.001 to 0.1 weight percent of non-ionic surfactant. Suitable non-ionic surfactants include polyoxyethylene ethers (BRIJ such as lauryl, cetyl, oleyl, stearyl, and tridecyl polyoxyethylene ethers; polyoxyethylene sorbitans (TWEEN such as polyoxyethylene sorbital monolaurate, monopalmitate, monostearate, monoleate, and trioleates); and other polyoxyethylene ethers (TRITON, for example). A preferred non-ionic surfactant is octylphenoxypolyethoxy ethanol having 40 ethylene oxide units (TRITON X-405, Rohm and Hass Company).

The insoluble support is then contacted with labeled antibody which will bind with antibodies conjugated to the insoluble support. Since a majority of anti-plaque antibodies are IgG antibodies, labeled anti-IgG antibodies can be used. If the detection of antibodies of the IgA and IgM classes are desired, the respective labeled anti-IgA and anti-IgM antibodies can be employed, along or in combination with the anti-IgG antibodies.

A variety of labels have been described above. For purposes of clarity and not by way of limitation, the subsequent steps of the process will be described for anti-IgG antibodies which have been labeled with an enzyme, preferably a fluorogenic enzyme. The term "fluorogenic enzyme" is defined herein to refer to an enzyme which will produce a fluorophore product with a suitable substrate.

The enzyme labeled anti-IgG antibody is applied to the insoluble support in an aqueous solution. The solution preferably contains suitable salts and buffers to preserve the reactants and facilitate the conjugation reaction. For example, the solution can contain bovine serum albumin (BSA), phosphate buffer solution (PBS), and a mild surfactant such as polyoxyethylene sorbitan ester employed in the above-described rinse solution. The incubation is continued for sufficient time to permit labeled anti-Ig antibody to conjugate with immunoglobulin, if any, adhering to the insoluble support. The preferred incubation times and temperatures are as set forth for the conjugation of serum anti-plaque antibody with the plaque reagent bound to the insoluble support.

The labeled anti-Ig solution is then removed form the insoluble support, and the support is rinsed with a rinse solution such as is describe above, to remove any residual, unconjugated labeled material.

In a third step of the sandwich process of this invention, the insoluble support is contacted with an aqueous solution of a substrate which undergoes a reaction in the presence of the enzyme to release fluorescent compound into the solution. Suitable substrates and the enzymes which which they can be converted are described in U.S.Patents 4,190,496 and 4,528,267, for example. The support is contacted with an aqueous solution of the substrate containing from $10^{-2}$ to $10^{-10}$ molar concentrations of the substrate. Substrate molar concentrations of from $10^{-4}$ to $10^{-5}$ are preferred. Preferred additional reagents and buffers in the substrate solution include 2-amino-2-methyl-1-propanol buffer and magnesium chloride, for example.

The substrate solution is incubated with the insoluble support for sufficient time for the reaction yielding the fluorophore to occur. At temperatures of from 18- 40C, incubation times of from 5 to 240 minutes can be used. Preferably, the temperature is within the range of from 20-26C, and the incubation time is from 30 to 90 minutes.

The fluorescent level in the solution is then measured. The equipment and procedures for determining the level of fluorescence in the substrate solutions are those conventionally used in the art. The level of fluorescence is a function of the enzyme concentration on the insoluble support which is, in turn, a function of the amount of anti-plaque antibody in the serum sample. The concentration of the anti-plaque antibody can be determined by comparing the fluorescence level of the solution with fluorescence levels obtained with control solutions containing known concentrations of anti-plaque antibody.

In another embodiment of this invention, the level of anti-plaque antibody in the serum sample is obtained by a competition immunoassay. In one embodiment of this invention, an insoluble support having a known concentration of plaque antigen is used. A mixture of the serum sample and a known concentration of labeled anti-plaque reagent antibody is applied to the insoluble support for a time sufficient to permit anti-plaque conjugation with plaque to occur. The times and temperatures described above for anti-plaque conjugation with plaque are suitable. After removing and rinsing the residual serum solution from the insoluble support, the label remaining on the insoluble support or in the serum solution mixture can be determined. If the label is a fluorogenic enzyme, the amount of label on the insoluble support can be determined by contacting the insoluble support with a suitable substrate solution, permitting the reaction yielding the fluorophore in the solution, and measuring the fluorescence level, as described above with respect to the sandwich immunoassay embodiment.

In another embodiment of a competition immunoassay, the insoluble support has anti-plaque reagent antibody adhered thereto. A mixture of the serum sample and labeled plaque antigen is then incubated with the insoluble support for a time sufficient to permit anti-plaque antibody to con]usage with plaque antigen. The amount of label on the insoluble support or remaining in serum solution is then determined. If the label is a fluorogenic enzyme, the amount of label on the insoluble support can be determined by contacting the insoluble support with a suitable substrate solution, permitting the reaction yielding the fluorophore in the solution, and measuring the fluorescence level, as described above with respect to the sandwich immunoassay embodiment.

This invention is further illustrated by the following specific, but non-limiting examples. Temperatures are given in degrees Centigrade and percents as weight percents unless otherwise specified. Examples which are constructively reduced to practice herein are presented in the present tense, and examples representing laboratory experiments previously reduced to practice are presented in the past tense.

Example 1

Isolating Atherosclerotic Plaque Antigen

The intimal layer (plaque) is stripped from the medial layer, and washed briefly in 0.9 N saline. The plaque is cut into small pieces (2×2 mm), and the tissue is homogenized in 0.15 M PBS at pH 7.2 at high speed for 30-60 sec. at 4C. The homogenate is centrifuged (2000 × g) at 4C for 30 min. The supernatant is saved. The homogenizing and centrifuging steps are repeated, pooling the supernatants. Sodium azide in bacteriostatic quantities is added to the pooled supernatant, and the supernatant is stored at 4C.

Example 2

Antibody Conjugation to Sepharose

Freeze-dried CNBr-Sepharose 4B powder (Pharmacia) is swelled for 15 min in 1 mM HCl. The gel is washed on a sintered glass filter (porosity G-3) with a total of 200 ml of 1 mM HCL per gram of gel (dry weight). This is done in several aliquots, the supernatant being suctioned off between successive additions.

Five mg of protein to be coupled per 1 ml of gel is dissolved in Coupling Buffer (0.1 M NaHCO3, pH 8.3, containing 0.5 M NaCl). The gel is washed with Coupling Buffer, the excess is removed by suction, and the protein solution is mixed with the gel. The mixtures is allowed to stand overnight at 4C without stirring. The gel is then placed in a Blocking Buffer containing 1 M ethanolamine, pH 8.0, for 2 hr at room temp. The gel is then washed with the Coupling Buffer, 0.1 M Acetate Buffer, pH 4.0, containing 0.5 M NaCl, and washed twice with the Coupling Buffer. The protein-Separose conjugate is now ready for use and can be stored at 4-8C. Cyanogen bromide can be added to the buffer solution as a bacteriostat.

Example 3

IgG Antibody Adsorption from Plaque Supernatant

A column is packed with 25 ml of Sepharose gel conjugated to anti-IgG antibody prepared in accordance with the procedure of Example 2, containing a total of about 130 mg of anti-IgG antibody. The column is equilibrated with 2-3 volumes of buffer (0.15 M PBS at pH 7.2), and the sample is then applied to the column.

The flow rate of eluting buffer (0.15 M PBS at pH 7.2) is 25 ml/hr. The eluted fractions containing antibody are collected until peak activity disappears.

The column is then washed with 10 × volumes of 0.15 PBS buffer, pH 7.2.

Additional desorption is obtained by eluting further with Acetate buffer, pH 4.0, at a flow rate of 25 ml/hr. The eluted peak samples are dialyzed against 0.15 M PBS, pH 7.2, for 24-36 hrs at 4C with multiple buffer changes.

The column is then washed with distilled water to desorb immunoaffinity bound IgG antibody. HPLC grade distilled water is perfused through the column in a volume equal to the void volume and elution is stopped for 6 hrs. The column is then eluted with additional distilled water at a rate of 15-20 ml/hr, collecting the eluted samples and retaining peak fractions. The peak fractions are dialyzed against 0.15 M PBS, pH 7.2 for 24-36 hrs at 4C with multiple buffer changes.

Example 4

IgA Antibody Adsorption from Plaque Supernatant

The procedure of Example 3 is repeated with a column packed with 7.5 ml of Sepharose gel conjugated to anti-IgE antibody prepared in accordance with the procedure of Example 2. The flow rate of eluting PBS buffer is 25 ml/hr.

Example 5

IgE Antibody Adsorption from Plaque Supernatant

The procedure of Example 3 is repeated with a column packed with 7.5 ml of Sepharose gel conjugated to anti-IgA antibody prepared in accordance with the procedure of Example 2. The flow rate of eluting PBS buffer is 25 ml/hr.

## Example 6

### IgM Antibody Adsorption from Plaque Supernatant

The procedure of Example 3 is repeated with a column packed with 10.0 ml of Sepharose gel conjugated to anti-IgM antibody prepared in accordance with the procedure of Example 2. The flow rate of eluting PBS buffer is 25 ml/hr.

## Example 7

### Plaque Antigen Purification

IgG antibody binding specifically with plaque obtained in accordance with the procedures of Example 3 is bound to Sepharose in accordance with the procedure of Example 2 to form an affinity column gel binding specifically to plaque antigen.

A column is packed with 25 ml of Sepharose gel conjugated to anti-plaque IgG antibody. The column is equilibrated with 2-3 volumes of buffer (0.15 M PBS at pH 7.2), and plaque solution obtaining according to the procedure of Example 1 is applied to the column.

The flow rate of eluting buffer (0.15 M PBS at pH 7.2) is 25 ml/hr. The eluted fractions containing plaque are collected until peak activity disappears, yielding plaque to which the anti-plaque IgG antibody specifically binds.

The column is then washed with 10 × volumes of 0.15 PBS buffer, pH 7.2.

Repeating this procedure but replacing the gel conjugated with anti-plaque IgG antibodies with gel conjugated with anti-plaque IgA, IgE and IgM antibodies obtained in accordance with the procedures of Examples 4, 5 and 6, respectively, yields the corresponding plaque fractions to which the antibodies specifically bind.

## Example 8

### Plaque Antigen Conjugation to BSA Carrier

A 0.01 M PBS solution, pH 6.8, of plaque antigen obtained in accordance with the procedure of Example 7 is prepared and cooled in an ice bath. Ten microliters of 5% BSA is added per ml of antigen solution, and 5 mg of 1-ethyl-3-(3-N,N-dimethylaminopropyl)carbodiimide (ECDI) is added to the mixture with stirring, and incubated at 4C for 20 min. The addition of ECDI and incubation is repeated three additional times. The mixture is maintained at 4C overnight with stirring, and the reaction mixture is dialyzed against 0.01 M PBS, pH 6.8, with multiple buffer changes for 48 hrs.

## Example 9

### Plaque Antigen Coated Microtiter Plate Preparation

100 Microliters of prepared dilutions of plaque antigen-BSA conjugate solution prepared in accordance with the procedures of Example 8 are applied to the surface of IMMULON II microtiter plates (Dynatech). The coating solution dilutions are 1:10, 1:100, 1:1000 and 1:10,000. The plates are tapped gently and to insure the coating solution covers the bottom of each well completely. The wells are incubated at 4C overnight in a covered, humidified box.

The coating solution is discarded and 200 microliters of 1% BSA in PBS is added per well. The wells are then incubated at room temperature for 1 hr in a humidity box, and the BSA solution is removed. The wells are then washed with 200 microliters of Wash Buffer (PBS, 0.5% Tween and 0.02% sodium azide), and stored in a humidity box at 4C until use.

Example 10

Fluorescence Immunoassay Procedure for Anti-(plaque antigen) IgG antibodies

To each microtiter plate coated with a different concentration of plaque antigen-BSA conjugate (prepared with plaque antigen purified with an affinity column conjugated with anti-plaque IgG antibody) is applied 100 microliters/well of affinity purified goat anti-IgG antibody solution (1:3000 dilution). The plates are covered to prevent drying and incubated for 2 hrs, and the residual goat anti-IgG antibody solution is removed, washing the plates three times with Wash Buffer.

In duplicate, 100 microliters/well of the serum sample of a patient being tested is applied to microtiter wells. The plates are covered to prevent drying and incubated for 2 hrs. The sera is removed and the plate is washed three times with Wash Buffer.

100 Microliters/well of affinity purified goat anti-IgG alkaline phosphatase conjugated antibody solution (1:3000 dilution) is applied to each well, and the plates are covered to prevent drying and incubated for 2 hrs. The anti-IgG solution is removed, and the plates are washed three times with Wash Buffer.

100 Microliters/well of 4-methyl-umbelliferyl phosphate solution (3M Diagnostic Systems) is then applied to the well. The microtiter plates are then read in a fluorometer (3M Diagnostics) every 10 minutes until the first 4096 reading or 1 hr mark is reached. The readings are continued every 30 min for another 2 hrs.

Example 11

Fluorescence Immunoassay Procedure for Anti-plaque antigen) IgM antibodies

To each microtiter plate coated with a different concentration of plaque antigen-BSA conjugate (prepared with plaque antigen purified with an affinity column conjugated with anti-plaque IgM antibody) is applied 100 microliters/well of affinity purified goat anti-IgM antibody solution (1:3000 dilution). The plates are covered to prevent drying and incubated for 2 hrs, and the residual goat anti-IgM antibody solution is removed, washing the plates three times with Wash Buffer.

In duplicate, 100 microliters/well of the serum sample of a patient being tested is applied to microtiter wells. The plates are covered to prevent drying and incubated for 2 hrs. The sera is removed and the plate is washed three times with Wash Buffer.

100 Microliters/well of affinity purified goat anti-IgM alkaline phosphatase conjugated antibody solution (1:3000 dilution) is applied to each well, and the plates are covered to prevent drying and incubated for 2 hrs. The anti-IgM solution is removed, and the plates are washed three times with Wash Buffer.

100 Microliters/well of 4-methyl-umbelliferyl phosphate solution (3M Diagnostic Systems) is then applied to the well. The microtiter plates are then read in a fluorometer (3M Diagnostics) every 10 minutes until the first 4096 reading or 1 hr mark is reached. The readings are continued every 30 min for another 2 hrs.

Example 12

Fluorescence Immunoassay Procedure for Anti-(plaque antigen) IgA antibodies

To each microtiter plate coated with a different concentration of plaque antigen-BSA conjugate (prepared with plaque antigen purified with an affinity column conjugated with anti-plaque IgA antibody) is applied 100 microliters/well of affinity purified goat anti-IgA antibody solution (1:3000 dilution). The plates are covered to prevent drying and incubated for 2 hrs, and the residual goat anti-IgA antibody solution is removed, washing the plates three times with Wash Buffer.

In duplicate, 100 microliters/well of the serum sample of a patient being tested is applied to microtiter wells. The plates are covered to prevent drying and incubated for 2 hrs. The sera is removed and the plate is washed three times with Wash Buffer.

100 Microliters/well of affinity purified goat anti-IgA alkaline phosphatase conjugated antibody solution (1:3000 dilution) is applied to each well, and the plates are covered to prevent drying and incubated for 2 hrs. The anti-IgM solution is removed, and the plates are washed three times with Wash Buffer.

100 Microliters/well of 4-methyl-umbelliferyl phosphate solution (3M Diagnostic Systems) is then applied to the well. The microtiter plates are then read in a fluorometer (3M Diagnostics) every 10 minutes until the first 4096 reading or 1 hr mark is reached. The readings are continued every 30 min for another 2 hrs.

Example 13

Fluorescence Immunoassay Procedure for Anti-(plaque antigen) IgE antibodies

To each microtiter plate coated with a different concentration of plaque antigen-BSA conjugate (prepared with plaque antigen purified with an affinity column conjugated with anti-plaque IgE antibody) is applied 100 microliters/well of affinity purified goat anti-IgA antibody solution (1:3000 dilution). The plates are covered to prevent drying and incubated for 2 hrs, and the residual goat anti-IgE antibody solution is removed, washing the plates three times with Wash Buffer.

In duplicate, 100 microliters/well of the serum sample of a patient being tested is applied to microtiter wells. The plates are covered to prevent drying and incubated for 2 hrs. The sera is removed and the plate is washed three times with Wash Buffer.

100 Microliters/well of affinity purified goat anti-IgE alkaline phosphatase conjugated antibody solution (1:3000 dilution) is applied to each well, and the plates are covered to prevent drying and incubated for 2 hrs. The anti-IgE solution is removed, and the plates are washed three times with Wash Buffer.

100 Microliters/well of 4-methyl-umbelliferyl phosphate solution (3M Diagnostic Systems) is then applied to the well. The microtiter plates are then read in a fluorometer (3M Diagnostics) every 10 minutes until the first 4096 reading or 1 hr mark is reached. The readings are continued every 30 min for another 2 hrs.

EXAMPLE 14

Isolating Plaque Antigen

Human aortas are obtained at autopsy from patients who have died of myocardial infarction, cerebral vascular accident or from surgical procedures. The aortae are dissected free of surrounding tissue and fat and rinsed in saline to remove contaminating blood. Segments of plaques are separated and processed.

The plaque areas are removed and cut into small pieces of 2 x 2 mm. The tissue is washed briefly in saline and homogenized in isotonic phosphate-buffered saline (PBS) at pH 7.0-7.2 in a WARING BLENDER at high speed for 30 sec at 4C. The homogenate is centrifuged (2000 x g) for 30 min at 4C and eluted. The eluates from this and subsequent steps are saved and concentrated.

The sedimented debris is washed in cold PBS and spun repeatedly as above until the supernatant is clear and contains no chemically or immunochemically detectable protein. At his stage, over 95% of soluble protein have been removed. The sediment is then washed in distilled water and extracted with 0.02 M citrate buffer, pH 3.2 with constant stirring at 37C for 2 hr. After this elution procedure, the mixture is centrifuged (2000 × g) for 30 min. The supernatant fraction is neutralized with 0.5 M NaOH and dialyzed overnight against PBS. The supernatant as well as the pooled PBS eluates are concentrated by vacuum dialysis and ultrafiltration to yield a concentrate of soluble plaque antigens.

For isolation of insoluble plaque antigens, portions of the aorta plaque are digested with different enzymes, the digest is extracted with PBS, and the eluates are concentrated as described above. The enzyme compositions used include collagenase [100 units/ml] in 0.05 M Tris buffer, pH 7.4 containing 5 mM $CaCl_2$; elastase [20 units/ml] in 0.035 M carbonate buffer, pH 8.8 containing 0.2% NaCl; DNase [500 units/ml] in 0.1 M monosodium phosphate buffer, pH 7.0 containing 2.5 mM $MgCl_2$; hyaluronidase [50 units/ml] in 0.1 M monosodium phosphate, pH 5.3 buffer containing 0.15 M NaCl; and neuraminidase [1 unit/ml] in modified Ringer's solution, pH 7.3 containing 2 mM EDTA. 10 Ml of digestion mixture is used per gram of aorta plaque. Each tissue-enzyme mixture is incubated at 37C in a shaking water bath. The duration of incubation is 24 hr for collagenase and elastase, 15 hr for hyaluronidase and 1 hr for DNase and neuraminidase. Each digest is centrifuged (2000 × g), and the liquid portion is concentrated to 0.5-1.0 with AMICON ultrafiltration membranes (PM 10), dialyzed against 0.15 M PBS for 24 hours to yield a concentrated solution of insoluble plaque antigens.

EXAMPLE 15

Isolating Anti-Plaque Antibodies

A portion of the soluble extract obtained according to the procedure of Example 1 is processed to separate autoantibodies therefrom. The concentrate is diluted with 0.15 M sodium chloride to contain approximately 15 mg./ml of protein including anti-plaque antibodies. The proteins are precipitated with anhydrous sodium sulfate (18 g/100 ml) made at 25C. After one hour, the precipitate is collected by centrifugation (10,000 × g, 20 min, 25C) and dissolved in 0.8 volu. of 0.1 M PBS, pH 7.5. The material is reprecipitated again as described. The sediment is dissolved in 0.1 M tris-HCl buffer (pH 8.0, 20C) and applied to a column (3.2 × 30 cm) of DEAE-SEPHADEX A-50 (Pharmacia) equilibrated with the same buffer. Elution of the material is performed by a continuous gradient from 0.1 M tris-HCl at a constant pH of 8.0 at 20C. Fractions containing IgA, IgD, IgE, IgG and IgM immunoglobulins are collected and concentrated. They are then applied to a column (3.2 × 95 cm) of SEPHADEX G-150 (Pharmacia) equilibrated with 0.1 M tris-HCl, 0.2 M NaCl, 0.002 M $EDTANa_2$, pH 7.7 containing 0.2% sodium azide. The material was allowed to pass through the column three times (recycling chromatography technique). Fractions containing IgA, IgD, IgE, IgG and IgM immunoglobulins are collected and concentrated.

EXAMPLE 16

Isolating Purified Plaque Antigens

The plaque antigens obtained according to the procedure of Example 2 is purified by affinity chromatography according to the procedure of Mishell and Shilgi in SELECTED METHODS IN CELLULAR IMMUNOLOGY. San Francisco: Freeman (1980), the entire contents of which are hereby incorporated by reference. A cross-linked agarose gel which has been substituted with sothiocyanatophenoxyhydroxypropyl groups is suspended in an aqueous solution of sodium bicarbonate, and to this suspension is added one ml of a mixed purified anti-plaque antibody solution obtained in accordance with the procedure of Example 2. The mixture is incubated for 3 days at room temperature while being gently stirred. The particles are then centrifuged and washed twice each of 0.5 M $NaHCO_3$, 0.1 M acetate buffer, and 0.1 M tris buffer containing 1 wt.% BSA.

The antibody bonded particles are mixed with a solution of plaque antigen concentrate prepared in accordance with Example 1 in PBS. The mixture is incubated for 8 hrs (overnight) at 25C. The particles are then separated from the mixture and rinsed three times with PBS, pH 7.2, to remove residual unbound plaque antigen. The particles are then mixed with an excess of 2.5 M NASCN solution, pH 8.0, and incubated for 8 hrs at 25C. The solution containing affinity purified plaque antigen is then concentrated by negative pressure dialysis or ultrafiltration, and the plaque antigen solution is stored at 4C.

EXAMPLE 17

Poyclonal Anti-Plaque Reagent Antibody Preparation

Rabbits are injected intramuscularly with a mixture of 0.5 mg of plaque antigen prepared by the procedure of Example 3 in 0.2 ml of 0.15 M sodium chloride solution and 0.8 ml of complete Freund's adjuvant. The immunization is repeated for 14 days and then each week for 3 weeks. After a further 10 days have passed, blood is removed from the rabbits, and antiserum is recovered from the blood by allowing it to coagulate and removing the clot.

EXAMPLE 18

Enzyme Labeled Anti-Plaque Reagent Antibody

Anti-plaque reagent antibody prepared in accordance with the procedures of Example 5 is conjugated with alkaline phosphatase following the modified procedure of M. O'Sullivan et al, _Analytical Biochem._ 100:100 (1979), the entire contents of which are hereby incorporated by reference.

EXAMPLE 19

Enzyme Labeled Plaque Antigen

Repeating the procedure of Example 6 but replacing the antibody reagent with the plaque antigen obtained in accordance with the procedure of Example 3 yields alkaline phosphatase labeled plaque antigen.

**Claims**

1. Atherosclerotic plaque antigen substantially free from impurities.

2. Atherosclerotic plaque antigen of Claim 1 conjugated with a distinctive label.

3. Atherosclerotic plaque antigen of Claim 2 wherein the label is an enzyme, radioactive label, fluorophore, chromophore or metal.

4. Atherosclerotic plaque antigen of Claim 3 wherein the label is an enzyme.

5. Atherosclerotic plaque antigen of Claim 3 wherein the label is alkaline phosphatase.

6. Antibody which binds preferentially to atherosclerotic plaque antigen, the antibody being substantially free from impurities.

7. Antibody of Claim 6 conjugated with a distinctive label.

8. Antibody of Claim 7 wherein the label is an enzyme, radioactive label, fluorophore, chromophore or metal.

9. Antibody of Claim 8 wherein the label is an enzyme.

10. Antibody of Claim 9 wherein the label is alkaline phosphatase.

11. An insoluble support having adhering thereto, atherosclerotic plaque antigen of Claim 1.

14

12. An insoluble support having adhering thereto, antibody of Claim 6 which binds preferentially to atherosclerotic plaque antigen.

13. The insoluble support of Claim 12 wherein the insoluble support is a microwell.

14. A method for determining the presence of anti-(atherosclerotic plaque antigen) antibody in a serum sample comprising

a) Contacting the sample with an insoluble support to which atherosclerotic plaque antigen is adhered for a time sufficient to permit plaque antigen conjugation with anti-(atherosclerotic plaque antigen) antibody to occur;

b) Contacting the insoluble support from step (a) with primary antibody which will bind with anti-(atherosclerotic plaque antigen) antibody for a sufficient time to permit conjugation to occur therebetween; and

c) Determining the primary antibody conjugated to the insoluble support.

15. The method of Claim 14 wherein the primary antibody is an anti-(human IgG) antibody.

16. The method of Claim 14 wherein the primary antibody is an anti-(human IgA) antibody.

17. The method of Claim 14 wherein the primary antibody is an anti-(human IgG) antibody.

18. The method of Claim 14 wherein the primary antibody is an anti-(human IgE) antibody.

19. The method of Claim 14 wherein the primary antibody has a distinctive label, and step (c) comprises determining the label bound to the insoluble support.

20. The method of Claim 17 wherein the label is an enzyme.

21. The method of Claim 18 wherein step (c) comprises contacting the insoluble support with a substrate which in the presence of the enzyme, produces a detectable chromophore or fluorophore.

22. The method of Claim 19 wherein the enzymne is alkaline phosphatase.

Claims for the following Contracting States: AT, GR, ES.

1. A method of preparing a reagent useful in immunoassays, comprising purifying human arteriosclerotic plaque to yield atherosclerotic plaque antigen substantially free from impurities.

2. Method according to claim 1 comprising conjugating the antigen with a distinctive label.

3. Method according to claim 2 wherein the label is an enzyme, radioactive label, fluorophore, chromophore or metal.

4. Method according to claim 3 wherein the label is an enzyme.

5. Method according to claim 3 wherein the label is alkaline phosphatase.

6. A method of preparing an antibody which binds preferentially to atherosclerotic plaque antigen, the antibody being substantially free from impurities, comprising immunizing an animal with plaque antigen and separating the immunoglobulins from serum from the animal.

7. Method of claim 6 including conjugating the antibody with a distinctive label.

8. Method of claim 7 wherein the label is an enzyme, radioactive label, fluorophore, chromophore or metal.

9. Method of claim 8 wherein the label is an enzyme.

10. Method of claim 9 wherein the label is an alkaline phpsphatase.

11. A method of preparing an insoluble support for use in immunoassay comprising adhering to the support atherosclerotic plaque antigen made according to claim 1.

12. A method of preparing an insoluble support for use in immunoassay comprising adhering to the support antibody made according to claim 6 which binds preferentially to atherosclerotic plaque antigen.

13. Method according to claim 11 or claim 12 wherein the insoluble support is a microwell.

14. A method for determining the presence of anti-(atherosclerotic plaque antigen) antibody in a serum sample comprising

a) Contacting the sample with an insoluble support to which atherosclerotic plaque antigen is adhered for a time sufficient to permit plaque antigen conjugation with anti-(atherosclerotic plaque antigen) antibody to occur;

b) Contacting the insoluble support from step (a) with primary antibody which will bind with anti-(atherosclerotic plaque antigen) antibody for a sufficient time to permit conjugation to occur therebetween; and

c) Determining the primary antibody conjugated to the insoluble support.

15. The method of Claim 14 wherein the primary antibody is an anti-(human IgG) antibody.

16. The method of Claim 14 wherein the primary antibody is an anti-(human IgA) antibody.

17. The method of Claim 14 wherein the primary antibody is an anti-(human IgG) antibody.

18. The method of Claim 14 wherein the primary antibody is an anti-(human IgE) antibody.

15

19. The method of Claim 14 wherein the primary antibody has a distinctive label, and step (c) comprises determining the label bound to the insoluble support.

20. The method of Claim 17 wherein the label is an enzyme.

21. The method of Claim 18 wherein step (c) comprises contacting the insoluble support with a substrate which in the presence of the enzyme, produces a detectable chromophore or fluorophore.

22. The method of Claim 19 wherein the enzymne is alkaline phosphatase.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | US-A-4 343 734 (J.B. LIAN et al.)<br>* Whole document *<br>--- | 1-3,6-8 | C 07 K 15/00<br>C 12 P 21/00<br>A 61 K 39/00<br>G 01 N 33/564<br>G 01 N 33/68 |
| X | EP-A-0 189 688 (RESEARCH DEVELOPMENT CORP. OF JAPAN)<br>* Whole document * | 1,6-9 | |
| A | <br>--- | 10,15-18 | |
| Y | EP-A-0 094 603 (AMERICAN HOECHST CORP.)<br>* Abstract; page 2, lines 1-14; page 7, line 24 - page 10, line 3 * | 11,13-15,17,19-22 | |
| A | <br>--- | 6-10 | |
| X | BIOLOGICAL ABSTRACTS, vol. 69, no. 10, 1980, page 6690, abstract no. 62690, Philadelphia, PA, US; W. HOLLANDER et al.: " Soluble proteins in the human atherosclerotic plaque: With spectral reference to immunoglobulins, c omplement C-3, x1-antitrypain and x2-macroglobulin", & ATHEROSCLEROSIS 34(4): 391-406, 1979<br>* Abstract * | 1 | |
| Y | Idem | 11,13-15,17,19-22 | |
| Y | US-A-4 493 899 (L.H. SMITH et al.)<br>* Abstract; column 1, lines 41-60; column 4, line 63 - column 5, line 5 *<br>--- -/- | 11,13-15,17,19-22 | |

TECHNICAL FIELDS SEARCHED (Int. Cl.4)

G 01 N
A 61 K

The present search report has been drawn up for all claims

| Place of search | Date of completion of the search | Examiner |
|---|---|---|
| THE HAGUE | 09-02-1988 | HITCHEN C.E. |

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| A | BIOLOGICAL ABSTRACTS, vol. 80, no. 11, 1985, page 437, abstract no. 95651, Philadelphia, PA, US; R. VLAICU et al.: "Quantitative determinations of immunoglobulins and complement components in human aortic atherosclerotic wall", & REV ROUM MED MED INTERNE 23(1): 29-36. 1985 | 1 | |
| | --- | | |
| E | EP-A-0 236 637 (RESEARCH DEVELOPMENT CORP. OF JAPAN) * Whole document * | 1,6 | |
| | ----- | | |

TECHNICAL FIELDS
SEARCHED (Int. Cl.4)

The present search report has been drawn up for all claims

| Place of search | Date of completion of the search | Examiner |
|---|---|---|
| THE HAGUE | 09-02-1988 | HITCHEN C.E. |